# EUROPÄISCHE PATENTSCHRIFT

(11) **EP 1 039 859 B1**
(45) Veröffentlichungstag und Bekanntmachung des Hinweises auf die Patenterteilung: **03.12.2003**
(21) Anmeldenummer: 98964494.3
(22) Anmeldetag: 09.12.1998
(51) Int. Cl.: A61F 5/56

(54) **IMPLANTAT ZUR IMPLANTATION IN EINE ZUNGE**
IMPLANT FOR IMPLANTATION IN THE TONGUE
IMPLANT DESTINE A ETRE IMPLANTE DANS UNE LANGUE

(30) Priorität: 20.12.1997 DE 19756956
(43) Veröffentlichungstag der Anmeldung: 04.10.2000
(73) Patentinhaber: Fege, Wolfgang, 33014 Bad Driburg (DE)
(72) Erfinder: Fege, Wolfgang, 33014 Bad Driburg (DE)
(86) Internationale Anmeldenummer: EP9807990
(87) Internationale Veröffentlichungsnummer: WO99032057

(56) Entgegenhaltungen:
- WO-A-92/09249
- DE-U- 29 603 998

## Beschreibung

Die Erfindung betrifft ein Implantat zur Implantation in eine Zunge.

Bei dem obstruktiven Schlafapnoesyndrom, im folgenden wie auch in der Fachliteratur kurz OSAS genannt, treten in den Tiefschlafphasen und REM-Schlafphasen (REM = Rapid-Eye-Movement) wiederkehrende Atempausen auf, in denen eine muskuläre Entspannung und als Folge davon ein Zurückfallen insbesondere des Zungenkörpers aufgrund der Schwerkraft auftritt. Der Zungenkörper legt sich dann im Extremfall an die Rachenhinterwand an und verschließt hierdurch den Luftweg in diesem Bereich.

Diese Atempausen enden erst dann, wenn durch die Sauerstoffentsättigung des Blutes und den Sauerstoffmangel im Gehirn ein plötzliches Aufwecken erfolgt, bis eine flachere Schlafstufe oder vollständiges Erwecken erreicht wird, wobei die muskuläre Grundspannung wieder so weit zunimmt, daß sich der Zungenkörper von der Rachenhinterwand zurückzieht und den Atemweg wieder freigibt. Danach mündet der Schlaf langsam wieder in eine REM- oder tiefere Schlafphase, bis der oben beschriebene Vorgang erneut beginnt.

Das OSAS ist behandlungsbedürftig, weil durch die während des Schlafens regelmäßig wiederkehrenden Sauerstoffentsättigungen des Blutes Herzmuskelschäden, Herzkranzgefäßerkrankungen, Hirngefäßerkrankungen mit Häufung von Schlaganfall und Hirnverkleinerung sowie Bluthochdruckkrisen und eine Vielzahl von hormonellen Störungen eintreten können. Zudem führt das häufige Ausbleiben der Tiefschlafphasen, die Störung der REM-Schlafphasen und das häufige Erwecken zu Antriebslosigkeit, Tagesmüdigkeit und leichter Einschlafneigung während des Tages, was beim Bedienen von Maschinen oder beim Autofahren zu Unfällen führen kann.

Es ist bekannt, daß OSAS durch Erzeugen eines konstanten Überdruckes im Nasen-Rachen-Raum mittels eines extrakorporalen Kompressors während des Schlafes über einen Schlauch und eine Nasenmaske zu behandeln. Eine derartige Behandlung mit Überdruck kann auf Dauer zu Erweiterungen der Lungenbläschen und zu Verkürzungen der Lungenbläschen-Zwischenwände, dem sogenannten Lungenemphysem, führen und als Folge davon zu einer Verkleinerung der Gesamtlungen-Diffusionsfläche und zu Atemnot. Außerdem besteht der Nachteil, daß sich bei einem Lungenüberdruck die Pumpleistung der rechten Herzkammer erhöhen muß, um die gleiche Menge Blut gegen den erhöhten Gegendruck in die Lunge drücken zu können. Dies kann zu Muskelerkrankungen des rechten Herzens führen. Ein weiterer Nachteil besteht darin, daß das Tragen einer Maske während des Schlafs störend ist.

Durch das Buch von L. Grothe, H. Schneider "Schlafapnoe- und kardiovaskuläre Erkrankungen", Thieme 1996, sind orale Prothesen bekannt, die vor dem Einschlafen in den Rachen einzusetzen sind und den Zweck haben, den Zungengrund nach vorn zu drücken. Solche oralen Prothesen sind jedoch nur bei leichtgradigen Ausprägungen des OSAS erfolgversprechend und werden außerdem wegen der komplizierten Handhabung und der Unbequemlichkeiten nur von einem geringen Prozentsatz der Patienten toleriert.

Zur Behandlung des OSAS sind auch chirurgische Therapiemaßnahmen bekannt, die im wesentlichen in einer Verbesserung des Luftdurchlasses im Nasen- und Nasen-Rachen-Bereich bestehen. Es ist sogar auch versucht worden, Unter- und Oberkiefer oder ein ausgesägtes Unterkieferknochenviereck im Kinnbereich mit daran anhaftenden Zungenmuskeln vorzuverlagern, um so den Luftdurchlaß zu verbessern. Dies ist ein großer operativer Eingriff, der eine beträchtliche Belastung für den Patienten darstellt und darüber hinaus selten akzeptable Resultate liefert, da er, genauso wie die zuvor geschilderten chirurgischen Therapiemaßnahmen, nur indirekt behandelt, weil das Zurückfallen des Zungenkörpers und dessen Anlegen an die Rachenhinterwand und der damit verbundene Verschluß des Atemweges nicht ursächlich vermieden wird.

Der Erfindung liegt die Aufgabe zugrunde, ein Implantat zu einer bisher noch nicht bekannten Implantation in eine Zunge anzugeben, durch das das OSAS wirksam vermieden oder verringert wird und Beeinträchtigungen des Patienten wie Schluckbeschwerden und Störungen während des Schlafs vermieden werden.

Die der Erfindung zugrunde liegende Aufgabe wird durch die im Patentanspruch 1 angegebene Lehre gelöst.

Der Grundgedanke dieser Lehre besteht darin, ein Implantat zu schaffen, das so geformt und ausgebildet ist, daß es unmittelbar in die Zunge so implantierbar ist, daß es den Zungengrund gegen ein Zurückfallen abstützt und so einen Luftdurchtritt auch bei erschlaffter Zunge bzw. Zungengrund sicherstellt. Durch die Möglichkeit der Implantation entfällt ein Anbringen oder Entfernen durch den Patienten. Außerdem besteht grundsätzlich die Möglichkeit, das Implantat so anzuordnen und auszubilden, daß der Schluckvorgang nicht oder nur unwesentlich beeinträchtigt wird.

Bei dem erfindungsgemäßen Implantat sind zwei Funktionen erfüllt. Zur Befestigung und/oder zur Abstützung am Zungenbein und/oder am/im seitlich von der Zunge gelegenen Gewebe dient ein Befestigungsteil, während zu der grundlegenden Abstützung des Zungengrundes ein Stützteil dient, das sich von dem Befestigungsteil in den Bereich des Zungengrundes und wenigstens teilweise quer zur Richtung der Zunge erstreckt.

Das Stützteil kann in verschiedener Form ausgebildet sein. Eine zweckmäßige Ausführungsform des Stützteils besteht darin, daß es so gekrümmt ist, daß im implantierten Zustand die konkave Seite dem Rachen zugewandt ist. Durch diese Form wird die Bildung eines Luftdurchlasses zwischen Zungengrund und Rachenhinterwand begünstigt.

Eine andere zweckmäßige Ausführungsform des Stützteils besteht in einer Laschenform, wobei sich die Lasche im implantierten Zustand im wesentlichen senkrecht zur Zunge erstreckt. Bei dieser Ausführungsform ist es zweckmäßig, daß das Befestigungsteil durch eine Verbreiterung des Fußes des laschenförmigen Stützteils gebildet ist. Diese Verbreiterung kann zweckmäßigerweise die Form von zwei flachen Vorsprüngen haben, die sich im implantierten Zustand zu beiden Seiten des Implantats bis in den Bereich des Zungenbeines erstrekken. Die Vorsprünge können sich dabei an dem Zungenbein abstützen, und außerdem können sie durch geeignete Mittel, z.B. Schrauben, am Zungenbein befestigt werden und einwachsen.

Eine Weiterbildung dieser Ausführungsform besteht darin, daß zwischen den beiden Vorsprüngen ein dritter flacher Vorsprung angeordnet ist, der im implantierten Zustand in Richtung auf das Zungenbein weist und so in einen in das Zungenbein gefrästen Schlitz implantierbar ist. Er kann dort zusätzlich durch Stifte befestigt werden oder in bei Implantaten bekannter Weise einwachsen.

Ist das Befestigungsteil durch zwei flache Vorsprünge gebildet, so können diese klammerartig ausgebildet sein, derart, daß sie im implantierten Zustand das Zungenbein wenigstens teilweise umklammern. Dies hat den Vorteil, daß zusätzliche Befestigungsmittel oder Maßnahmen nicht erforderlich sind.

Das Stützteil kann die Form eines Streifens haben, der im implantierten Zustand zur Rachenhinterwand hin konkav gebogen ist und dessen Enden sich im implantierten Zustand an der Rachenhinterwand und/oder dem seitlichen Rachengewebe abstützen und so das Stützteil bilden. Zweckmäßigerweise besteht dabei der Streifen aus flexiblem Material. Dies hat zur Folge, daß der Schluckvorgang nur wenig beeinträchtigt wird, während andererseits die konkave Biegung des streifenförmigen Stützteils eine staumauerartige Wirkung entfaltet, so daß trotz hoher Flexibilität des Materials, was in bezug auf das Schlucken günstig ist, verhältnismäßig große Staukräfte durch das Stützteil aufgefangen und so wirksam ein Zurücksinken des Zun-gengrundes vermieden werden kann.

Auch bei den anderen Ausführungsformen des Stützteils kann dieses aus flexiblem Material bestehen, wobei die Flexibilität in fachmännischer Weise so zu wählen ist, daß einerseits die Bewegungen der Zunge, insbesondere beim Schlucken, nicht oder nicht wesentlich behindert werden, andererseits die Flexibilität aber nicht so groß ist, daß die Stützwirkung verlorengeht.

In vorteilhafter Weise können das Stützteil und das Befestigungsteil aus körpereigenem Material bestehen. Das körpereigene Material kann durch Entnahme aus dem Körper des Patienten gewonnen werden. Beispielsweise kann dafür ein Rippenknorpel verwendet werden, der entsprechend geformt worden ist. Eine andere zweckmäßige Möglichkeit besteht darin, das körpereigene Material durch einen vorgeformten gezüchteten Rohling zu bilden. Entweder kann dieser Rohling unmittelbar die Form des gewünschten Implantats haben. Es ist aber auch möglich, das Implantat durch Bearbeitung eines Rohlings zu bilden, der noch nicht die Form des Implantats hat.

Bei allen vorhergehenden Ausführungsformen ist es zweckmäßig, daß das Implantat eine rauhe Oberfläche, insbesondere Vorsprünge und/oder nach außen offene Kanäle aufweist. Dadurch wird das Einwachsen des Implantats unterstützt. Die Kanäle können außerdem in der Weise zum Einheilen verwendet werden, daß sie, z.B. zu einem gemeinsamen Ausgang am unteren Implantatende zusammengeführt und dann mit einer Unterdruckquelle, z.B. mit einer Saugdrainage, verbunden werden, so daß so nicht nur Blut und Wundsekret abgesaugt, sondern auch das Implantat umgebendes Gewebe jedenfalls teilweise in die Kanäle eingesaugt wird, um so eine innige Einheilung herbeizuführen. Außerdem kann in den oder die Kanäle später bei Bedarf Röntgenkontrastmittel injiziert werden, um den Sitz des Implantats zu prüfen oder Implantatrisse festzustellen, die durch Austreten des Kontrastmittels sichtbar werden.

Die Vorsprünge und/oder Vertiefungen können grundsätzlich in beliebiger Weise ausgebildet sein. Eine zweckmäßige Ausbildungsform besteht darin, daß sie durch ein das Implantat umgebendes Gewebe gebildet sind. Als Gewebe wird zweckmäßigerweise ein solches verwendet, von dem eine gute Einheilung, keine Resorption und selten Fremdkörperreaktionen bekannt sind.

Eine Weiterbildung der Erfindung besteht darin, daß das Implantat einen elastischen Kern aufweist, der von weichem Material umgeben ist. Der Kern bewirkt eine Stabilisierung der Form, während das weiche Material mechanisch den Übergang zu dem umgebenden Gewebe verbessert. Als Material für den elastischen Kern ist die Verwendung von Silikonen zweckmäßig. Es ist aber auch möglich, den Kern oder sogar das gesamte Implantat aus implantationsfähigen Metall herzustellen.

Anhand der Zeichnung soll die Erfindung an Ausführungsbeispielen näher erläutert werden.
- Fig. 1: zeigt ein Ausführungsbeispiel eines erfindungsgemäßen Implantats von oben,
- Fig. 2: zeigt das Implantat gemäß Fig. 1 von hinten,
- Fig. 3: zeigt das Implantat gemäß Fig. 1 von vorn,
- Fig. 4: zeigt das Implantat gemäß Fig. 1 in perspektivischer Darstellung in Verbindung mit einem Zungenbein,
- Fig. 5: zeigt ein zweites Ausführungsbeispiel der Erfindung,
- Fig. 6: zeigt ein drittes Ausführungsbeipiel der Erfindung,
- Fig. 7: zeigt ein viertes Ausführungsbeispiel der Erfindung,
- Fig. 8: ist ein schematischer Schnitt durch einen Kopf und verdeutlicht die Anbringung des Implantats gemäß Fig. 5 am Zungenbein,
- Fig. 9: zeigt perspektivisch ein weiteres Ausführungsbeispiel der Erfindung,
- Fig. 10: verdeutlicht die Implantationslage des Implantats gemäß Fig. 9 im Zungengrund,
- Fig. 11: ist ein schematischer Schnitt durch einen Kopf im Bereich einer Zunge mit einem Implantat gemäß Fig. 9 und
- Fig. 12: zeigt ein Ausführungsbeispiel ähnlich gemäß Fig. 5.

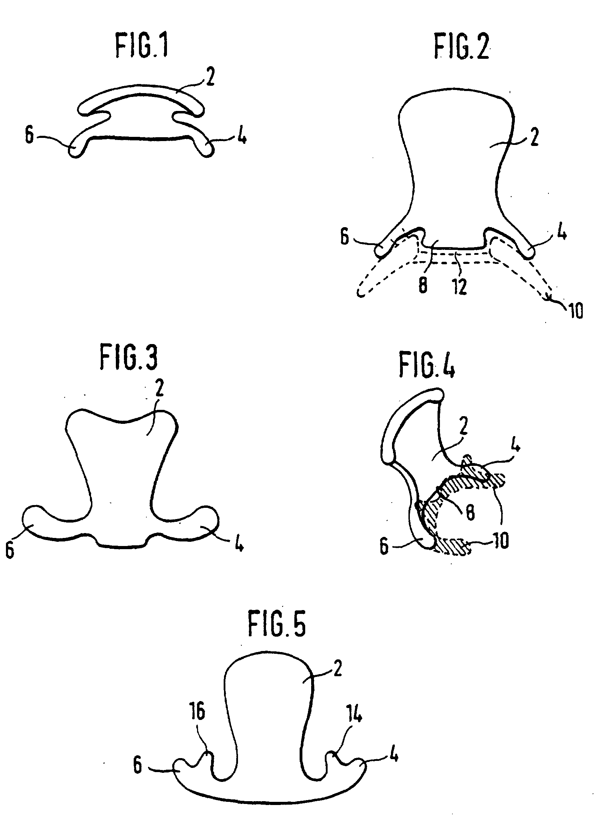

Fig. 1 zeigt ein Implantat gemäß der Erfindung von oben, das ein gekrümmtes Stützteil 2 und ein Befestigungsteil in Form von zwei seitlichen Vorsprüngen 4 und 6 aufweist. Die Implantation dieses Implantats erfolgt in der Weise, daß die konkave Seite des gekrümmten Stützteils zum Rachen hin weist.

Fig. 2 zeigt das Implantat gemäß Fig. 1 von hinten, wobei durch gestrichelte Linien ein Zungenbein angedeutet ist. Zwischen den beiden Vorsprüngen 4 und 6 befindet sich ein weiterer Vorsprung 8, der, wie das gesamte Implantat, im wesentlichen flach ausgebildet ist und dazu dient, in einen in ein mit gestrichelten Linien angedeutetes Zungenbein 10 eingefrästen Schlitz 12 eingesetzt zu werden.

Fig. 3 zeigt die Ausführungsform des Implantats gemäß Fig. 1 und 2 von vorn.

Fig. 4 zeigt das Implantat gemäß den Fig. 1 bis 3 in perspektivischer Darstellung, wobei, wie in Fig.2, das Zungenbein mit gestrichelten Linien angedeutet ist. Es wird, genauso wie in Fig. 2, deutlich, daß die seitlichen Vorsprünge 4 und 6 sich auf dem Zungenbein abstützen, während der Vorsprung 8 im mittleren Bereich in einen gefrästen Schlitz im Zungenbein 10 ragt.

Fig. 5 zeigt in ähnlicher Darstellung wie Fig. 2 ein weiteres Ausführungsbeispiel der Erfindung, das im wesentlichen mit dem Ausführungsbeispiel gemäß Fig. 2 übereinstimmt. Gleiche oder sich entsprechende Teile sind mit gleichen Bezugsziffern versehen. Der Unterschied besteht darin, daß der zentrale Vorsprung 8 gemäß Fig. 2 fehlt, während zusätzlich an den seitlichen Vorsprüngen 4 und 6 Nasen 14 und 16 angeordnet sind, die im implantierten Zustand das Zungenbein wenigstens teilweise bündig umschließen sollen, um so die Abstützung der durch die Vorsprünge 4 und 6 gebildeten Stützteile zu verbessern.

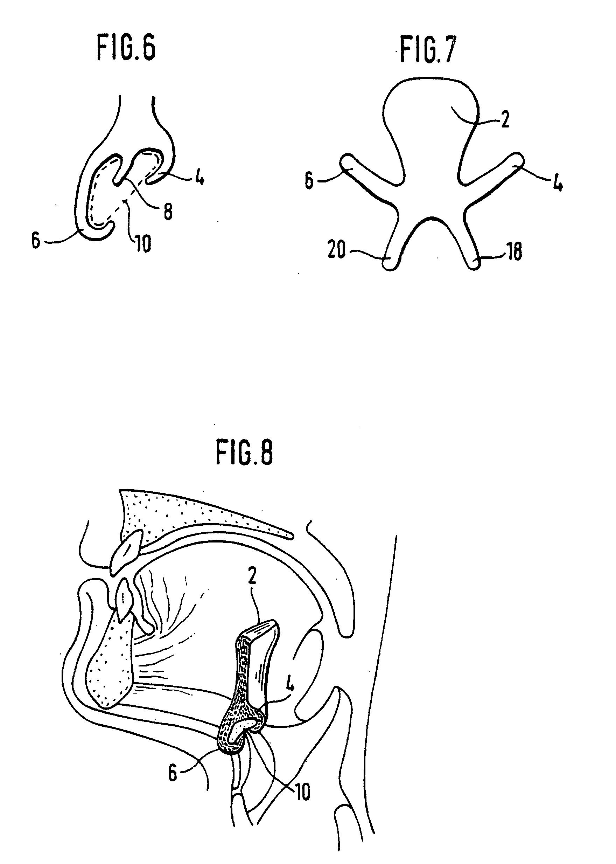

Fig. 6 zeigt den unteren Teil eines weiteren Ausführungsbeispiels der Erfindung ähnlich gemäß Fig. 1 bis 4. Gleiche oder sich entsprechende Teile sind mit gleichen Bezugsziffern versehen. Unterschiede bestehen darin, daß die seitlichen Vorsprünge 4 und 6 sowie der mittlere Vorsprung 8 langgestreckt ausgebildet sind, so daß sie in der Lage sind, im implantierten Zustand benachbarte Teile des Zungenbeines 10 zu umschließen.

Fig. 7 zeigt ein Ausführungsbeispiel ähnlich gemäß Fig. 1 bis 4. Gleiche oder sich entsprechende Teile sind mit gleichen Bezugsziffern versehen. Der Unterschied besteht darin, daß der mittlere Vorsprung 8 weggelassen und stattdessen Füßchen 18 und 20 vorgesehen sind, die das Implantat im implantierten Zustand unter oder an dem Schildknorpel des Kehlkopfes abstützen.

Fig. 8 verdeutlicht die Anbringung des Implantats gemäß Fig. 6. Es ist deutlich zu erkennen, daß die Vorsprünge 4 und 6 das Zungenbein 10 klammerartig umgreifen.

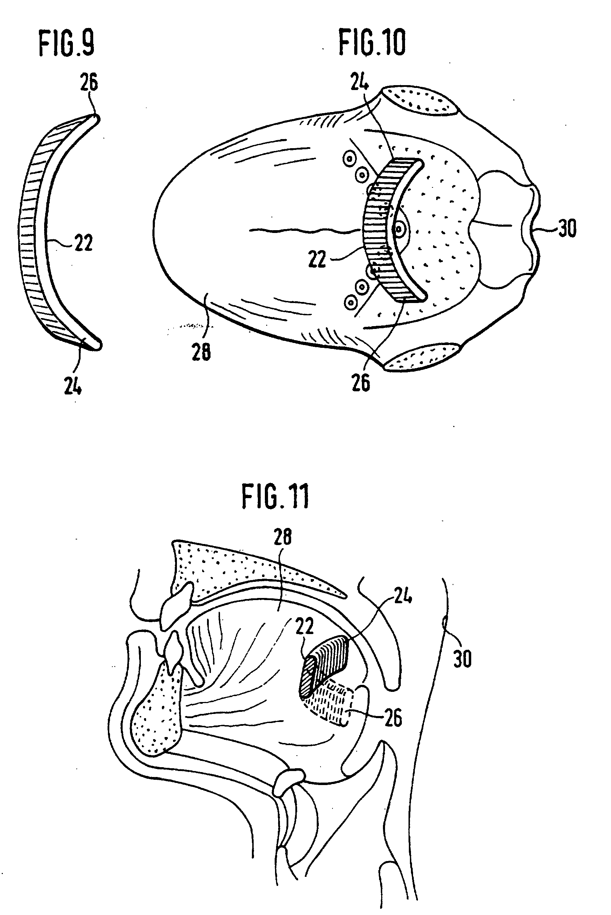

Fig. 9 zeigt ein Ausführungsbeispiel einer grundsätzlichen anderen Ausführungsform der Erfindung in perspektivischer Darstellung. Das Stützteil hat die Form eines Streifens 22, der gebogen ist und so zu implantieren ist, daß die Biegung im implantierten Zustand zur Rachenhinterwand hin konkav ist. Die Länge des Streifens 22 ist so bemessen, daß Enden 24 und 26 sich im implantierten Zustand an der Rachenhinterwand oder seitlich am und/oder im Gewebe abstützen und so das Stützteil bilden.

Dies wird anhand der Fig. 10 und 11 deutlich, die schematisch das Implantat gemäß Fig. 9 im implantierten Zustand zeigen.

Fig. 10 zeigt eine Draufsicht auf eine Zunge 28 eines Patienten mit umgebenden Teilen und mit in die Zunge 28 implantierten Implantat gemäß Fig. 9. Es ist zu erkennen, daß die konkave Seite des Implantats zu einer Rachenhinterwand 30 weist. Der Streifen 22 besteht aus einem Material, das flexibel ist. Das bedeutet, daß der Streifen 22 staumauerartig den Zungengrund gegen ein Zurückfallen zur Rachenhinterwand 30 hin abstützt, wobei die Stützkräfte durch die Enden 24 und 26 auf das seitliche Gewebe übertragen werden. Von besonderem Vorteil bei dieser Ausführungsform ist es, daß durch die Flexibilität in Verbindung mit der Biegung des Streifens 24 der Schluckvorgang nicht beeinträchtigt wird.

Fig. 11 zeigt einen Schnitt durch einen Kopf im Bereich der Zunge 28, in deren Zungengrund der Streifen 22 implantiert ist.

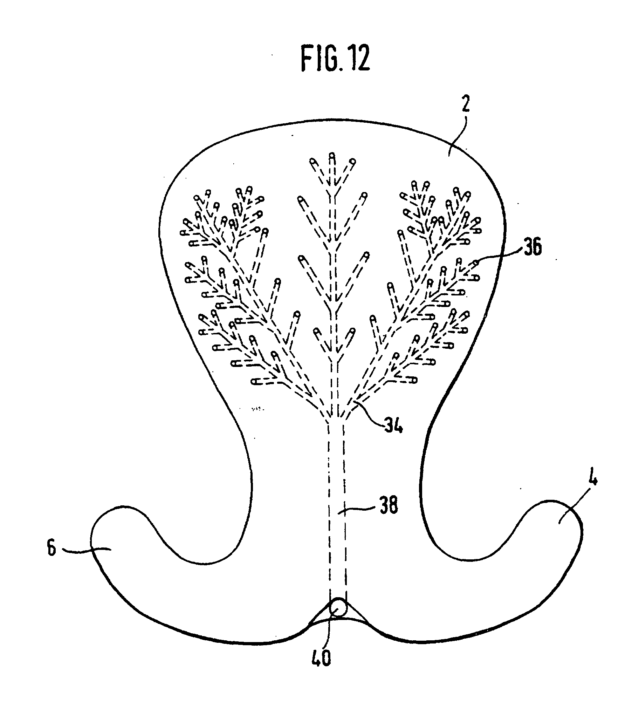

Fig. 12 zeigt ein Ausführungsbeispiel ähnlich Fig. 5. Gleiche oder sich entsprechende Teile sind mit gleichen Bezugsziffern versehen. Der Unterschied besteht darin, daß in dem Stützteil 2 Kanäle 34 vorgesehen sind, die sich verästeln und zu Öffnungen 36 führen und über einen Kanal 38 mit einem gemeinsamen Ausgang 40 verbunden sind, der an eine Saugdrainage angeschlossen werden kann, durch die nicht nur Blut und Wundsekret abgesaugt, sondern auch das Implantat umgebendes.Gewebe jedenfalls teilweise in die Öffnungen 36 eingesaugt werden kann, um so eine innige Einheilung herbeizuführen.

## Patentansprüche

1. Implantat zur Implantation in eine Zunge,
mit einem Befestigungsteil (4, 6, 8) zur Befestigung und/oder zur Abstützung am Zungenbein (10) und/oder am/im seitlich von der Zunge gelegenen Gewebe, und
mit einem Stützteil (2), das sich von dem Befestigungsteil (4, 6, 8) in den Bereich des Zungengrundes und wenigstens teilweise quer zur Richtung der Zunge (28) erstreckt.

2. Implantat nach Anspruch 1, wobei das Stützteil (2) gekrümmt ist, derart, daß im implantierten Zustand die konkave Seite der Rachenhinterwand (30) zugewandt ist.

3. Implantat nach Anspruch 1, wobei das Stützteil (2) laschenförmig ausgebildet ist und sich im implantierten Zustand im wesentlichen senkrecht zur Zunge (28) erstreckt.

4. Implantat nach Anspruch 3, wobei das Befestigungsteil durch eine Verbreiterung des Fußes des laschenförmigen Stützteils gebildet ist.

5. Implantat nach Anspruch 4, wobei das Befestigungsteil durch zwei flache Vorsprünge (4, 6) gebildet ist, die sich im implantzierten Zustand zu beiden Seiten des Implantats bis in den Bereich des Zungenbeines (10) erstrecken.

6. Implantat nach Anspruch 5, wobei zwischen den beiden Vorsprüngen (4, 6) ein dritter Vorsprung (8) angeordnet ist, der im implantierten Zustand in Richtung auf das Zungenbein (10) weist und so in einen in das Zungenbein (10) gefrästen Schlitz (12) implantierbar ist.

7. Implantat nach Anspruch 5, wobei die beiden Vorsprünge (4, 6) klammerartig ausgebildet sind, derart, daß sie im implantierten Zustand das Zungenbein (10) wenigstens teilweise umklammern.

8. Implantat nach Anspruch 1, wobei das Stützteil die Form eines Streifens (22) hat, der im implantierten Zustand zur Rachenhinterwand (30) hin konkav gebogen ist und dessen Enden (24, 26) im implantierten Zustand sich seitlich am und/oder im Gewebe abstützen und so das Stützteil bilden.

9. Implantat nach Anspruch 8, wobei der Streifen (22) aus flexiblem Material besteht.

10. Implantat nach Anspruch 1, wobei das Stützteil (2) aus flexiblem Material besteht.

11. Implantat nach Anspruch 1, wobei das Stützteil (2) und das Befestigungsteil aus körpereigenem Material bestehen.

12. Implantat nach Anspruch 11, wobei das körpereigene Material durch Entnahme aus dem Körper des Patienten gewonnen ist.

13. Implantat nach Anspruch 11, wobei das körpereigene Material durch einen vorgeformten gezüchteten Rohling gebildet ist.

14. Implantat nach einem der vorgehenden Ansprüche, wobei das Implantat eine rauhe Oberfläche, insbesondere Vorsprünge und/oder Vertiefungen und/oder nach außen offene Kanäle aufweist.

15. Implantat nach Anspruch 14, wobei die Vorsprünge und/oder Vertiefungen durch ein das Implantat umgebendes Gewebe gebildet sind.

16. Implantat nach einem der vorgehenden Ansprüche, wobei in dem Implantat von außen zugängliche Kanäle angeordnet sind.

17. Implantat nach einem der vorgehenden Ansprüche, wobei das Implantat einen elastischen Kern aufweist, der von weichem Material umgeben ist.

## Claims

1. Implant for implantation in a tongue,
with a fixing part (4, 6, 8) for the fixing and/or for the support at the hyoid bone (10) and/or at/in the tissue situated lateral of the tongue, and
with a supporting part (2), which extends from the fixing part (4, 6, 8) in the area of the root of tongue and at least partly crosswise to the direction of the tongue (28).

2. Implant according to claim 1, in which the supporting part (2) is curved, in such a way, that in the implanted state the concave side is facing the back wall of the pharynx (30).

3. Implant according to claim 1, in which the supporting part (2) is formed strapshaped and in the implanted state extends in essence vertical to the tongue (28).

4. Implant according to claim 3, in which the fixing part is created by a widening of the foot of the strapshaped supporting part.

5. Implant according to claim 4, in which the fixing part is created by two flat projections (4, 6), which extend in the implanted state at both sides of the implant up to the area of the hyoid bone (10).

6. Implant according to claim 5, in which between the two projections (4, 6) a third projection (8) is arranged, which points at the hyoid bone (10) in the implanted state and so is implantable in a slit (12) milled in the hyoid bone (10).

7. Implant according to claim 5, in which the two projections (4, 6) are formed clamplike, in such a way, that they at least partially clasp the hyoid bone (10) in the implanted state.

8. Implant according to claim 1, in which the supporting part has the form of a strip (22) which in the implanted state is bent concave to the back wall of the pharynx (30) and whose ends (24, 26) in the implanted state support themselves lateral at and/or in the tissue and create the supporting part in this way.

9. Implant according to claim 8, in which the strip (22) is made of flexible material.

10. Implant according claim 1, in which the supporting part (2) is made of flexible material.

11. Implant according claim 1, in which the supporting part (2) and the fixing part are made of a material occuring naturally in the body.

12. Implant according to claim 11, in which the material occuring naturally in the body can be obtained by taking out of the body of the patient.

13. Implant according to claim 11, in which the material occuring naturally in the body is formed by a preshaped cultivated blank.

14. Implant according to one of the foregoing claims, in which the implant shows a rough surface, in particular projections and/or deepenings and/or outwardly open canals.

15. Implant according to claim 14, in which the projections and/or deepenings are made of a tissue surrounding the implant.

16. Implant according to one of the foregoing claims, in which canals being accessible from outward are arranged in the implant.

17. Implant according to one of the foregoing claims, in which the implant shows an elastic core, which is surrounded by soft material.

## Revendications

1. Implant destiné à l'implantation dans une langue,
avec une partie de fixation (4, 6, 8) servant à la fixation et / ou au soutien sur l'os hyoïde et / ou sur / dans le tissu situé latéralement par rapport à la langue, et
avec une partie d'appui (2) qui s'étend de la partie de fixation (4, 6, 8) jusqu'au niveau de la base de la langue, et ce au moins en partie transversalement par rapport au sens de la langue (28).

2. Implant selon la revendication 1, la partie d'appui (2) étant courbée de telle manière qu'à l'état implanté le côté concave soit tourné vers la paroi postérieure du pharynx (30).

3. Implant selon la revendication 1, la partie d'appui (2) étant constituée sous forme de patte et s'étendant à l'état implanté essentiellement de manière perpendiculaire à la langue (28).

4. Implant selon la revendication 3, la partie de fixation étant formée par un élargissement de la base de la partie d'appui en forme de patte.

5. Implant selon la revendication 4, la partie de fixation étant formée par deux saillies plates (4, 6) qui s'étendent à l'état implanté des deux côtés de l'implant jusqu'au niveau de l'os hyoïde (10).

6. Implant selon la revendication 5, une troisième saillie (8) étant disposée entre les deux saillies (4, 6), laquelle saillie étant dirigée à l'état implanté en direction de l'os hyoïde (10) et pouvant être ainsi implantée dans une fente (12) fraisée dans l'os hyoïde (10).

7. Implant selon la revendication 5, les deux saillies (4, 6) étant constituées en forme d'agrafes, de telle sorte qu'à l'état implanté elles enserrent au moins en partie l'os hyoïde (10).

8. Implant selon la revendication 1, la partie d'appui ayant la forme d'une bande (22) qui, à l'état implanté, est courbée de manière concave vers la paroi postérieure du pharynx (30) et dont les extrémités (24, 26) s'appuient, à l'état implanté, latéralement sur et / ou dans le tissu du pharynx, formant ainsi la partie d'appui.

9. Implant selon la revendication 8, la bande (22) étant constituée de matière flexible.

10. Implant selon la revendication 1, la partie d'appui (2) étant constituée de matière flexible.

11. Implant selon la revendication 1, la partie d'appui (2) et la partie de fixation étant constituées de matière endogène.

12. Implant selon la revendication 11, la matière endogène étant gagnée par prélèvement sur le corps du patient.

13. Implant selon la revendication 11, la matière endogène étant formée par une ébauche préformée et cultivée.

14. Implant selon l'une des revendications précédentes, l'implant présentant une surface rugueuse, notamment des saillies et / ou des renfoncements et / ou des canaux ouverts vers l'extérieur.

15. Implant selon la revendication 14, les saillies et / ou les renfoncements étant formés par un tissu entourant l'implant.

16. Implant selon l'une des revendications précédentes, des canaux accessibles depuis l'extérieur étant disposés dans l'implant.

17. Implant selon l'une des revendications précédentes, l'implant présentant un noyau élastique qui est entouré de matière souple.
